# FASCICULE DE BREVET EUROPEEN

(11) **EP 3 405 895 B1**
(45) Date de publication et mention de la délivrance du brevet: **10.03.2021**
(21) Numéro de dépôt: 17706543.0
(22) Date de dépôt: 19.01.2017
(51) Int. Cl.: G16H 20/17, A61M 5/172, A61B 5/145, A61B 5/00, G16H 50/20, G16H 20/10

(54) **GESTION DE L'ÉQUILIBRE GLYCÉMIQUE D'UN PATIENT DIABÉTIQUE**
MANAGEMENT DER BLUTZUCKERBALANCE EINES PATIENTEN MIT DIABETES
MANAGEMENT OF THE BLOOD GLUCOSE BALANCE OF A DIABETIC PATIENT

(30) Priorité: 21.01.2016 FR 1650483
(43) Date de publication de la demande: 28.11.2018
(73) Titulaire: Voluntis, 75017 Paris (FR)
(72) Inventeur: BAVIERE, Eric, 78125 Gazeran (FR); D'ORSAY, Geneviève, 75015 Paris (FR); MOUCHEROUD, Guillaume, 92170 Vanves (FR)
(74) Mandataire: Plasseraud IP
(86) Numéro de dépôt international: PCT/FR2017/050108
(87) Numéro de publication internationale: WO 2017/125682

(56) Documents cités:
- EP-A1- 2 862 586
- EP-A2- 2 628 115
- WO-A2-2014/209630
- US-A1- 2011 106 011
- US-A1- 2014 024 907
- Louis Monnier ET AL: "Glycemic Variability: The Third Component of the Dysglycemia in Diabetes. Is It Important? How to Measure It?", , 1 novembre 2008 (2008-11-01), XP055287098, Extrait de l'Internet: URL:http://www.ncbi.nlm.nih.gov/pmc/articl es/PMC2769808/pdf/dst-02-1094.pdf [extrait le 2016-07-08]

## Description

### Domaine technique et art antérieur

La présente invention concerne le domaine de la santé.

La présente invention concerne plus particulièrement les dispositifs médicaux logiciels adaptés pour améliorer l'accompagnement thérapeutique des patients diabétiques.

Un des objectifs de la présente invention est de concevoir un dispositif médical logiciel configuré pour détecter précocement le retour du patient dans un état de déséquilibre glycémique afin notamment d'améliorer l'état de santé général du patient et d'éviter que le patient se trouve dans un état d'hyperglycémie ou d'hypoglycémie pendant une durée de temps prolongée.

Le diabète est une maladie chronique liée à une absence ou une déficience de la production d'insuline ; en d'autres termes, un patient diabétique ne peut réguler correctement la quantité de sucre dans le sang.

On distingue principalement deux types de diabète :
- le diabète de type I, ou diabète insulino-dépendant (DID), qui est un diabète dans lequel le corps du patient ne fabrique plus d'insuline. Il concerne environ 350.000 personnes en France ;
- le diabète de type II, ou diabète non insulinodépendant (DNID), qui est un diabète dans lequel la production d'insuline est insuffisante ou déficiente. Il aboutit à une dégradation progressive de la capacité du patient à réguler son taux de sucre dans le sang. Le diabète de type II concerne plus de 2.500.000 personnes en France.

Chez le diabétique de type I, un traitement à base d'insuline, soit par injection, soit par pompe, est la seule thérapie possible.

Dans les stades précoces du diabète de type II, le traitement consiste essentiellement à accompagner le patient dans une modification de son mode de vie en agissant principalement sur son alimentation et la pratique d'une activité physique.

Dans une phase ultérieure, le patient reçoit ensuite un premier support médicamenteux par exemple sous la forme d'antidiabétiques oraux.

Puis, au fur et à mesure de l'évolution de la maladie et de la dégradation progressive de la fonction pancréatique du patient, celui-ci passe à un traitement par injection d'insuline.

Ce passage sous injection d'insuline est un changement important dans la vie du patient.

La peur de la piqûre, la crainte de l'hypoglycémie en cas de surdosage, les réactions du corps à ce nouveau traitement sont autant de paramètres qui complexifient le traitement thérapeutique du patient.

Il existe de nombreux protocoles médicaux permettant l'accompagnement du patient dans cette phase de mise sous insuline.

Dans l'ensemble de ces protocoles, les objectifs sont sensiblement les mêmes.

Les protocoles se concentrent exclusivement sur la phase dite de titration ; celle-ci consiste principalement à atteindre progressivement la dose d'insuline ciblée en :
- augmentant pas-à-pas la dose d'insuline prise par le patient pour éviter des effets indésirables liés à une augmentation trop rapide de l'apport d'insuline. Cette phase de titration utilise par exemple un algorithme de calcul reposant notamment sur la dernière glycémie du patient, l'insuline utilisée et la dernière dose recommandée, et la sensibilité à l'insuline du patient ;
- arrêtant cette augmentation dès lors que le taux de glycémie du patient atteint un objectif prédéterminé par son médecin. Cet objectif correspond sensiblement à un taux de glycémie se situant dans une plage de valeurs qui est généralement comprise entre 80 et 120 mg/dl.

Une fois que le taux de glycémie mesuré chez le patient se situe dans cette plage de valeurs, la dose du patient est réputée être à l'équilibre : on considère dès lors que le patient est dans un état d'équilibre glycémique. On parle également de phase d'équilibre glycémique.

Le Demandeur soumet ici qu'il est important pour des raisons médicales d'arrêter l'augmentation de la dose d'insuline une fois que le taux de glycémie est dans la plage de valeurs attendues.

Cette approche présente de nombreux inconvénients pour la santé du patient.

En effet, la phase de titration repose sur le dernier taux de glycémie mesuré et sur la dose recommandée précédemment pour déterminer l'évolution de la dose d'insuline journalière.

Les évènements passés (tels que par exemple l'activité physique, le stress, un oubli de la dernière injection, etc.) que le patient aurait oublié de prendre en compte ne sont pas considérés dans le calcul de la dose ; or, ces évènement peuvent directement influer sur la dose recommandée précédemment.

Si celle-ci a été surévaluée à cause d'un oubli du patient de déclarer un évènement hypoglycémique, le nouveau calcul de dose s'appuiera sur une dose surévaluée pour déterminer la nouvelle recommandation.

Par voie de conséquence, cette nouvelle dose pourra être trop élevée.

Une telle dose (trop élevée) n'est pas sans danger pour le patient : celle-ci peut précipiter l'occurrence d'une nouvelle hypoglycémie.

Le Demandeur soumet par ailleurs que les calculs de dose existants s'appuient sur la dernière dose recommandée, et non pas sur la dernière dose effectivement prise par le patient.

Ainsi, un patient ayant volontairement réduit sa dose par crainte d'une hypoglycémie se retrouvera assurément dans la période suivante avec un taux de glucose dans le sang trop élevé ; il se verra dès lors proposer d'augmenter sa dose d'insuline sans détecter que la cause principale du déséquilibre à ce stade est liée au sous dosage réalisé par le patient.

Dans ce cas, plutôt que de lui conseiller une nouvelle dose plus forte, il faut l'encourager à utiliser la dose effectivement recommandée.

De plus, les raisons pour lesquelles une glycémie à jeun du patient est juste en dessous du seuil maximal peuvent trouver plusieurs origines :
- les apports glucidiques du patient ont été réduits le jour précédent (par exemple le patient a moins mangé que d'habitude), ou parce que
- le patient a consommé plus de sucre le jour précédent (par exemple en pratiquant une activité physique particulière).

En tout état de cause, une glycémie à jeun du patient ne peut être juste en dessous du seuil maximal parce que le patient a atteint son point d'équilibre.

Or, les méthodes d'ajustement existantes ne permettent pas d'isoler et/ou de décider ne pas tenir compte d'événement exceptionnel pour retarder l'atteinte de l'équilibre insulinique.

Les conséquences de ce défaut d'interprétation sur l'interruption de la phase d'initiation de la titration sont que le patient reste ensuite déséquilibré jusqu'à sa prochaine visite chez son médecin, ce qui peut être préjudiciable pour sa santé.

Enfin, si un quelconque évènement vient perturber le métabolisme du patient, le patient peut rebasculer dans un état de déséquilibre.

Avec les méthodes existantes, le patient attend nécessairement sa prochaine visite chez le médecin pour réévaluer sa dose d'insuline ; il peut donc rester chroniquement en déséquilibre pendant une longue durée.

Le document US 2014/024907 A1 divulgue des méthodes permettant de détecter un état de déséquilibre glycémique d'un patient diabétique.

Le Demandeur soumet qu'il n'existe pas à ce jour dans l'état de la technique de solution permettant de détecter avec précision et de façon précoce l'apparition chez un patient diabétique d'un état de déséquilibre glycémique pendant une phase de stabilité.

### Résumé et objet de la présente invention

La présente invention vise à améliorer la situation actuelle décrite ci-dessus.

La présente invention permet de remédier aux différents inconvénients de l'état de la technique mentionnés ci-dessous en proposant une gestion personnalisée de l'équilibre glycémique d'un patient diabétique permettant notamment de détecter l'apparition d'un déséquilibre glycémique pendant une phase d'équilibre glycémique.

A cet effet, la présente invention concerne selon un premier aspect un dispositif médical de gestion de l'équilibre glycémique d'un patient diabétique.

Selon l'invention, le dispositif comporte un module mémoire qui est configuré pour mémoriser une pluralité de glycémies mesurées pendant une phase d'équilibre sur une période de temps déterminée.

Selon l'invention, ces glycémies mesurées sont relatives à une teneur d'un composant sanguin représentatif du taux de glycémie du patient.

Selon l'invention, le dispositif comporte en outre un circuit de traitement mettant en œuvre une règle de gestion configurée pour détecter un état de déséquilibre glycémique chez le patient et un circuit de titration configuré pour calculer une dose journalière d'insuline.

Selon l'invention, la règle de gestion prévoit une comparaison des glycémies mesurées avec une plage de valeurs seuil.

Selon l'invention, cette plage de valeurs seuil présente :
- une borne supérieure de glycémie correspondant à un état de glycémie élevée (par exemple une glycémie supérieure à 1,20 g/L à jeun correspondant sensiblement à un état d'hyperglycémie), et
- une borne inférieure de glycémie correspondant à un état de glycémie basse (par exemple une glycémie inférieure à 0,80 g/L à jeun correspondant sensiblement à un état d'hypoglycémie).

Selon l'invention, le circuit de traitement est configuré pour émettre un signal d'avertissement lorsqu'un état de déséquilibre glycémique est détecté.

Ainsi, grâce à cette combinaison de moyens techniques, caractéristique de la présente invention, on dispose d'un dispositif médical (par exemple un terminal de communication) simple d'utilisation permettant au patient diabétique d'être alerté ou d'alerter son médecin traitant lorsqu'un état de déséquilibre glycémique a été détecté.

De cette façon, le patient diabétique, qui, après une phase de titration, prend sa dose d'insuline cible et pense être en état d'équilibre glycémique, est alerté d'un déséquilibre dès son apparition.

Il peut dès lors prendre un rendez-vous chez son médecin traitant pour débuter une nouvelle phase de titration afin de ré-atteindre un état d'équilibre.

Ainsi, grâce à la présente invention et à cette détection précoce, le patient diabétique ne reste pas de façon prolongée dans un état glycémique.

Dans un mode de réalisation particulier, la règle de gestion, qui est mise en œuvre par le circuit de traitement, comprend un algorithme configuré pour détecter un état de déséquilibre glycémique chez le patient lorsqu'un pourcentage déterminé des valeurs de glycémie n'est pas dans la plage de valeurs seuil.

Selon un exemple d'implémentation possible, un état de déséquilibre glycémique est détecté chez le patient lorsque plus de 50% des valeurs de glycémie n'est pas dans la plage de valeurs seuil.

Selon un autre exemple d'implémentation, un état de déséquilibre glycémique est détecté chez le patient lorsque plus de 66% des valeurs de glycémie n'est pas dans la plage de valeurs seuil.

Dans un autre mode de réalisation particulier, la règle de gestion, qui est mise en œuvre par le circuit de traitement, comprend un autre algorithme configuré pour détecter un état de déséquilibre chez le patient lorsque la distribution des valeurs mesurées sur la période de temps présente un écart-type supérieur à une déviation seuil déterminée.

Avantageusement, le dispositif selon la présente invention comporte un processeur (ou circuit central) configuré pour, sur réception du signal d'avertissement :
- désactiver le circuit de traitement de manière à mettre fin à la phase d'équilibre, et
- activer un circuit de titration de manière à initialiser une (nouvelle) phase de titration permettant de déterminer une nouvelle dose journalière d'insuline.

Cette nouvelle phase de titration permet alors d'atteindre l'état d'équilibre. Selon l'invention, le circuit de titration est configuré pour émettre un signal d'équilibre lorsqu'un état d'équilibre glycémique est détecté au cours de la phase de titration, et le processeur est en outre configuré pour, sur réception du signal d'équilibre :
- activer le circuit de traitement, et
- désactiver le circuit de titration.

De préférence, le circuit de titration est apte à mettre en œuvre une règle de dosage configurée pour calculer ladite nouvelle dose journalière d'insuline en fonction notamment de valeurs de glycémie et de paramètres physiologiques et/ou médicaux propres au patient.

On comprend ici que les valeurs de glycémie utilisées pour réaliser ce calcul sont des valeurs mesurées pendant la phase de titration.

Ces mesures pourront être réalisées par exemple à l'aide d'une sonde glycémique.

Les paramètres physiologiques et/ou médicaux propres au patient sont quant à eux des informations saisies par le patient et/ou par le médecin traitant directement ou indirectement via ledit dispositif, ou sont des informations issues par exemple de mesures réalisées par des capteurs dédiés (activité, géolocalisation, etc.).

La règle de dosage selon l'invention peut également prendre en considération d'autres paramètres tels que par exemple une dose d'insuline cible telle que celle préconisée par le médecin lors d'une consultation préalable.

Dans un mode de réalisation particulier, la règle de dosage est configurée :
- pour diminuer la dose journalière d'au moins une unité lorsque, pendant une période d'au moins un jour, la moyenne des glycémies mesurées est strictement inférieure à la borne inférieure de glycémie, et
- pour augmenter la dose journalière d'au moins une unité lorsque, pendant une période d'au moins un jour, la moyenne des glycémies mesurées est strictement supérieure à la borne supérieure de glycémie.

Cette règle de dosage intègre une logique de calcul dans laquelle est réalisée une comparaison de la moyenne des glycémies mesurées sur une période de temps définie par rapport à une plage de valeurs déterminée.

On peut donc prévoir par exemple que la règle de dosage fait varier la dose d'insuline proportionnellement à l'écart entre cette moyenne et la plage de valeurs déterminée.

Alternativement, on peut prévoir que la règle de dosage peut utiliser des paliers en fonction de l'intervalle entre la moyenne et cette plage de valeurs déterminée. Dans ce cas, le nombre de paliers et les incréments propres à chaque palier sont configurables par le médecin.

En tout état de cause, cette règle de dosage permet après détection de l'état de déséquilibre d'amorcer une nouvelle phase dite de titration pour atteindre à nouveau l'équilibre glycémique.

Dans un mode de réalisation particulier, le dispositif comprend un circuit de contrôle configuré pour comparer la dose calculée avec la dose injectée dans le corps du patient.

On comprendra ici que la dose dite injectée dans le corps du patient peut être la dose réellement injectée ; cette dose est fournie par l'injecteur après injection (par exemple par asservissement). On peut par exemple envisager le cas d'un injecteur qui fournit cette information au circuit de titration une fois que la dose est injectée.

Alternativement, cette dose dite injectée est la dose déclarée par le patient après injection.

Avantageusement, le circuit de titration est apte à communiquer avec l'injecteur d'insuline pour récupérer les informations relatives à la dose réellement injectée dans le corps du patient de cette dose.

Avantageusement, le circuit de titration est apte à communiquer avec cet injecteur d'insuline pour transmettre au circuit de titration les informations relatives à la dose journalière d'insuline calculée de manière à injecter dans le corps du patient cette dose.

Avantageusement, le dispositif selon un mode de réalisation de la présente invention comporte au moins un capteur d'activité configuré pour mesurer et fournir au circuit de traitement des informations relatives à l'activité dudit patient.

Avantageusement, la règle de gestion est configurée pour traiter les informations d'activité fournies de manière à détecter au moins un événement perturbateur relatif à un changement d'activité dudit patient.

Avantageusement, la règle de gestion est configurée pour détecter un état de déséquilibre glycémique chez le patient en fonction dudit au moins un évènement perturbateur.

Dans un mode de réalisation particulier, ledit au moins un capteur comprend un capteur de mouvements.

Dans un autre mode de réalisation pouvant être combiné avec le précédent mode, ledit au moins un capteur comprend une sonde de géolocalisation apte à fournir des informations sur la localisation du patient.

De préférence, ledit au moins un capteur est couplé à une horloge interne.

Ceci permet de synchroniser les données collectées et mesurées.

Avantageusement, le dispositif selon un mode de réalisation particulier de l'invention comporte en outre une sonde glycémique configurée pour mesurer une teneur d'un composant sanguin représentatif du taux de glycémie dudit patient.

De préférence, la sonde glycémique est configurée pour communiquer les glycémies mesurées au module mémoire.

Avantageusement, la sonde glycémique est configurée pour réaliser les mesures à intervalle de temps régulier, par exemple tous les jours (éventuellement à heure fixe).

De préférence, le composant sanguin est le marqueur HbA1C, ou hémoglobine glyquée.

Avantageusement, le dispositif selon un mode de réalisation de l'invention comporte des moyens d'affichage configurés pour afficher les informations contenues dans le signal d'avertissement.

Un tel signal d'avertissement comprend par exemple l'information relative à l'état de déséquilibre glycémique détecté : par exemple une information relative à un état d'hyperglycémie ou une information relative à un état d'hypoglycémie.

Corrélativement, la présente invention concerne selon un deuxième aspect un procédé de gestion de l'équilibre glycémique d'un patient diabétique.

Selon l'invention, le procédé est mis en œuvre par des moyens informatiques et comporte les étapes suivantes :
- une mémorisation d'une pluralité de glycémies mesurées pendant une phase d'équilibre glycémique sur une période de temps déterminée, les glycémies mesurées étant relatives à une teneur d'un composant sanguin représentatif du taux de glycémie du patient ;
- une comparaison des glycémies mesurées avec une plage de valeurs seuil présentant une borne supérieure de glycémie correspondant à un état de glycémie élevée et une borne inférieure de glycémie correspondant à un état de glycémie basse ;
- une détection d'un état de déséquilibre glycémique chez le patient en fonction de la mise en œuvre d'une règle de gestion analysant les résultats de la comparaison ;
- en cas de détection d'un état de déséquilibre glycémique chez le patient, une émission d'un signal d'avertissement.

Avantageusement, lors de l'étape d'analyse des résultats de la comparaison, un état de déséquilibre glycémique est détecté chez le patient lorsqu'un pourcentage déterminé desdites glycémies mesurées n'est pas dans la plage de valeurs seuil.

Dans un mode de réalisation particulier, un état de déséquilibre glycémique est détecté chez le patient lors de l'étape d'analyse lorsque plus de 50% desdites glycémies mesurées n'est pas dans ladite plage de valeurs seuil.

Dans un autre mode de réalisation particulier, un état de déséquilibre glycémique est détecté chez le patient lors de l'étape d'analyse lorsque plus de 66% des glycémies mesurées n'est pas dans la plage de valeurs seuil.

Avantageusement, lors de l'étape d'analyse des résultats de comparaison, un état de déséquilibre glycémique est détecté chez le patient lorsque la distribution des glycémies mesurées sur la période de temps présente un écart-type supérieur à une déviation seuil déterminée. Cette déviation seuil est de préférence fonction de la plage de valeurs seuil.

Avantageusement, le procédé selon l'invention comprend, suite à la réception d'un signal d'avertissement, une étape d'arrêt de la phase d'équilibre glycémique suivie d'une étape d'initialisation de la phase de titration.

Selon l'invention, la phase de titration comprenant une étape de titration au cours de laquelle est mise en œuvre une règle de dosage pour déterminer une nouvelle dose journalière d'insuline

Selon l'invention, la phase de titration comporte, en cas de détection d'un état d'équilibre glycémique chez le patient, une émission d'un signal d'équilibre et, suite à la réception dudit signal d'équilibre, la désactivation du circuit de titration et l'activation du circuit de traitement.

Dans un mode de réalisation particulier, lors de l'étape de titration, la règle de dosage calcule la nouvelle dose journalière d'insuline en fonction des glycémies et de paramètres physiologiques et/ou médicaux propres au patient.

De préférence, lors de l'étape de titration, la règle de dosage calcule la dose journalière de la manière suivante :
- on diminue la dose journalière d'au moins une unité lorsque, pendant une période d'au moins un jour, la moyenne des glycémies mesurées est strictement inférieure à ladite borne inférieure de glycémie, et
- on augmente la dose journalière d'au moins une unité lorsque, pendant une période d'au moins un jour, la moyenne des glycémies mesurées est strictement supérieure à ladite borne supérieure de glycémie.

Le procédé peut également prévoir les autres cas déjà énoncés ci-dessus.

De préférence, le procédé comprend une communication des informations relative à la dose journalière d'insuline calculée à un injecteur d'insuline pour une injection de cette dose dans le corps du patient.

Le procédé selon un mode de réalisation de l'invention comprend avantageusement une première communication des informations relatives à la dose journalière d'insuline calculée à un injecteur d'insuline pour une injection de cette dose dans le corps du patient.

Le procédé peut également comprendre une deuxième communication des informations relatives à la dose réellement injectée dans le corps du patient par l'injecteur d'insuline.

De préférence, le procédé selon la présente invention comprend une étape de contrôle au cours de laquelle la dose calculée lors de l'étape de titration est comparée avec la dose injectée dans le corps du patient.

En effet, il est possible que la dose injectée corresponde à une dose déclarée par le patient. Dans ce cas, on comprend que cette dose déclarée peut être différente de la dose réellement injectée. Ceci permet au patient de choisir librement la dose qu'il s'injecte.

Le mode de réalisation proposé ci-dessus permet alors de prendre en considération cette déviation pour recalculer correctement la dose d'insuline le jour suivant.

Avantageusement, l'étape de détection comprend :
- une mesure de l'activité physique du patient,
- une détection d'au moins un événement perturbateur relatif à un changement d'activité du patient,

Dans ce cas, lors de l'étape d'analyse, la règle de gestion prend en considération ledit au moins un événement perturbateur pour détecter un état de déséquilibre glycémique chez le patient.

De préférence, le procédé selon un mode de réalisation de l'invention comprend une mesure d'une teneur d'un composant sanguin représentatif du taux de glycémie du patient.

De préférence, la mesure est réalisée à intervalle de temps régulier.

Dans un mode de réalisation avantageux, le composant sanguin est le marqueur HbA1C.

De préférence, le procédé selon un mode de réalisation de l'invention comprend un affichage des informations contenues dans le signal d'avertissement.

L'objet de la présente invention concerne également selon un troisième aspect un programme d'ordinateur comportant des instructions adaptées pour l'exécution des étapes du procédé tel que décrit ci-dessus, lorsque ledit programme d'ordinateur est exécuté par au moins un processeur.

Un tel programme d'ordinateur peut utiliser n'importe quel langage de programmation, et être sous la forme d'un code source, d'un code objet, ou d'un code intermédiaire entre un code source et un code objet, tel que dans une forme partiellement compilée, ou dans n'importe quelle autre forme souhaitable.

Bien évidemment, l'homme du métier comprendra ici que, par ordinateur, on pourra comprendre ici tout dispositif informatique comprenant un processeur (ou équivalent) apte à lire les instructions du programme et exécuter les étapes du procédé qui y sont associées.

Il pourra notamment s'agir un terminal de communication du type « Smartphone ».

On comprendra ici que le programme d'ordinateur permettra par exemple l'installation et la mise en œuvre d'une application logicielle sur le terminal de communication.

Le programme d'ordinateur selon l'invention peut être en particulier téléchargé sur un réseau de type Internet.

Ainsi, l'objet de la présente invention, par ses différents aspects fonctionnels et structurels décrits ci-dessus, permet de mettre à disposition des patients diabétiques un dispositif médical leur permettant d'être alerté (directement ou par l'intermédiaire de leur médecin traitant) de l'apparition d'un déséquilibre glycémique.

### Brève description des figures annexées

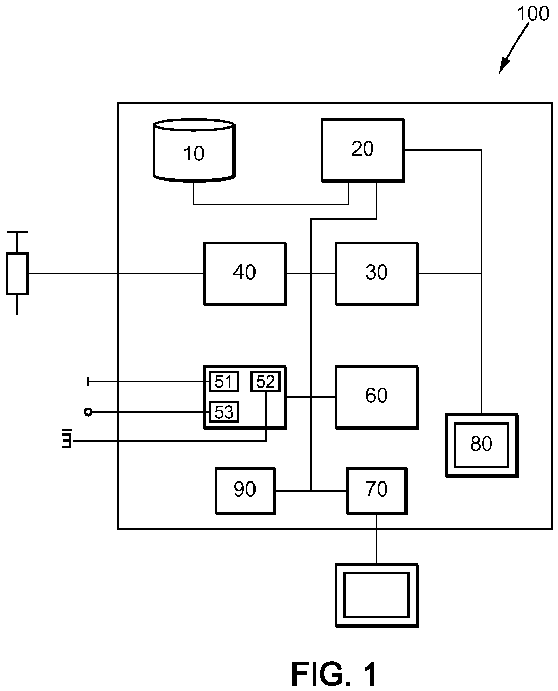

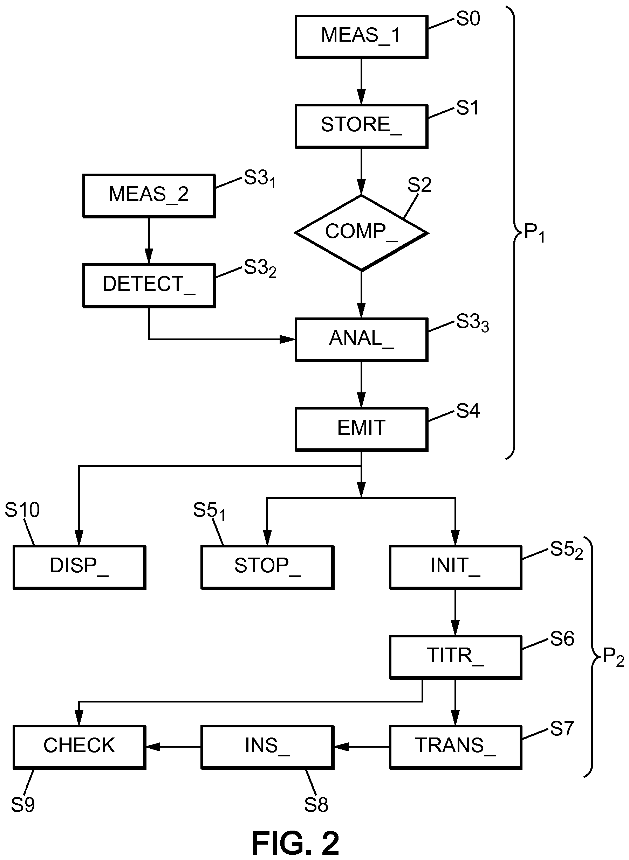

D'autres caractéristiques et avantages de la présente invention ressortiront de la description ci-dessous, en référence aux figures 1 et 2 annexées qui en illustrent un exemple de réalisation dépourvu de tout caractère limitatif et sur lesquelles :
- la figure 1 représente une vue schématique d'un dispositif gestion de l'équilibre glycémique d'un patient diabétique selon un exemple de réalisation de l'invention ;
- la figure 2 représente un organigramme des étapes du procédé de gestion selon un exemple de réalisation de la présente invention.

### Description détaillée de l'invention

La gestion et la surveillance de l'état glycémique d'un patient diabétique ainsi que le système qui lui est associé vont maintenant être décrits dans ce qui va suivre en faisant référence conjointement aux figures 1 à 2.

Pour mémoire, un des objectifs de la présente invention est de concevoir un dispositif médical logiciel comprenant des moyens informatiques et logiciels pour la gestion et la surveillance de l'équilibre glycémique d'un patient diabétique de type II afin notamment d'alerter le patient et/ou le médecin traitant en cas d'apparition d'un déséquilibre glycémique.

Ceci est rendu possible grâce à la présente invention qui sera décrite dans ce qui suit selon un exemple de réalisation particulier.

Dans cet exemple, le patient diabétique se rend chez son médecin traitant qui détermine selon les paramètres médicaux et/ou physiologiques du patient un taux de glycémie cible à atteindre.

Il lui prescrit ensuite un dispositif médical 100.

Un tel dispositif 100 est prévu pour assurer le suivi et l'accompagnement du patient dans son traitement.

Plus particulièrement, dans cet exemple, ce dispositif 100 est un terminal de communication sur lequel est installée une application médicale dédiée mettant en œuvre des fonctionnalités logicielles pour l'accompagnement du patient.

On parle également de dispositif d'ASG pour Auto-Surveillance Glycémique.

On sait que l'auto-surveillance glycémique chez un patient souffrant d'un diabète de type II est importante.

Cette auto-surveillance doit s'inscrire dans une démarche d'éducation du patient et de son entourage,
Lors de la prescription du dispositif 100 d'ASG, il est indispensable d'expliquer au patient les enjeux de son traitement et d'organiser avec lui cette auto-surveillance : fréquence, fixation des horaires, objectifs glycémiques, modifications du traitement à effectuer par le patient ou le médecin en fonction des résultats.

On sait par ailleurs que le patient doit avoir une alimentation et une activité physique adaptées.

Dans cet exemple, on se place dans le cas où le patient a atteint sa dose d'insuline dite d'équilibre ; on est dans ce cas dans une phase d'équilibre glycémique, noté P1.

Cette phase P1 fait donc suite à une titration préalable.

Comme cela est très souvent le cas, cette phase d'équilibre P1 n'est pas toujours durable : le diabète est évolutif et instable par nature. Le traitement doit donc être réévalué régulièrement dans toutes ses composantes.

Pour mémoire, un des objectifs de la présente invention est de détecter précocement l'apparition d'un déséquilibre glycémique.

A cet effet, le dispositif 100 comprend dans cet exemple une sonde glycémique 53 qui mesure lors d'une étape S0 une teneur d'un composant sanguin représentatif du taux de glycémie du patient.

De préférence, la mesure porte sur le marqueur HbA1C.

En effet, la plupart des stratégies médicamenteuses pour traiter le diabète de type II a retenu ce marqueur pour contrôler efficacement la glycémie du patient.

Dans le cas du marqueur HbA1C, la cible d'HbAlc inférieure ou égale à 7 % est recommandée. Le traitement médicamenteux doit être instauré ou réévalué si l'HbAlc est supérieure à 7 %.

D'autres marqueurs pourront être mesurés et analysés.

De préférence, cette mesure S0 se fait de façon périodique, par exemple tous les jours ou toutes les semaines.

Dans l'exemple décrit ici, la sonde glycémique 53 communique ensuite la ou les valeurs de glycémie mesurées à un module mémoire 10.

Ce module mémoire, intégré (ou non) dans le dispositif 100, stocke ces valeurs.

De préférence, le dispositif 100 comprend une horloge interne 60 qui permet d'horodater les glycémies mesurées et de faire en sorte que toutes les informations stockées dans le module mémoire 10 soient synchronisées entre elles.

Dans cet exemple, le dispositif 100 comporte ensuite un circuit de traitement 20 des glycémies mesurées.

Plus précisément, ce circuit 20 met en œuvre une règle de gestion qui va détecter un état de déséquilibre glycémique chez le patient en effectuant une comparaison S2 de chacune des glycémies mesurées et stockées sur le module mémoire 10 avec une plage de valeurs seuil déterminée.

De préférence, cette plage de valeurs présente une borne supérieure de glycémie correspondant sensiblement à un état d'hyperglycémie et une borne inférieure de glycémie correspondant sensiblement à un état d'hypoglycémie.

Plus particulièrement, la règle de gestion mise en œuvre sur le circuit de traitement 20 réalise une analyse S3₃ des résultats de la comparaison.

Dans cet exemple, la règle de gestion comprend un algorithme qui va détecter un état de déséquilibre glycémique chez le patient lorsque plus de 66% des valeurs de glycémie mesurées ne situe pas la plage de valeurs seuil.

Dans cet exemple, on comprend donc que la règle de gestion mise en œuvre sur le circuit 20 permet d'analyser la distribution des valeurs dans le temps.

Une distribution qui présente des variations élevées sera donc révélatrice de l'apparition d'un état de déséquilibre. Une telle analyse permet d'éviter une détection erronée qui serait dû par exemple à un évènement perturbateur.

Dans un mode de réalisation particulier, on prévoit également des capteurs tels que par exemple un capteur de mouvements 51 ou un capteur 52 de type GPS permettant de localiser le patient.

Les informations fournies par ces capteurs 51 et 52 pourront ainsi être traitées et prises en considération par la règle de gestion pour détecter l'apparition d'un état de déséquilibre.

En effet, une activité physique intense, un stress, un décalage horaire, une fatigue ponctuelle pourront influer directement sur la glycémie du patient.

La règle de gestion intègre donc la prise en compte de ces paramètres dans son algorithme pour éviter une détection erronée.

Lorsqu'un déséquilibre est détecté, le circuit de traitement 20 génère et émet à destination du processeur 80 un signal d'avertissement lors d'une étape S4.

Ce signal comprend notamment des informations sur le déséquilibre : par exemple un état d'hypoglycémie ou d'hyperglycémie.

Optionnellement, ces informations pourront être affichées lors d'une étape S10 sur un écran 70 du dispositif 100.

Dans l'exemple décrit ici, en cas de déséquilibre détecté, le processeur 80 reçoit donc le signal d'avertissement.

Sur réception de ce signal, il désactive lors d'une étape S5₁ le circuit de traitement 20.

Cette étape d'arrêt S5₁ met donc fin à la phase d'équilibre glycémique P1.

Le processeur 80 active ensuite lors d'une étape d'initialisation S5₂ un circuit de titration 30. Cette étape S5₂ permet de déclencher la phase de titration P2.

Cette phase P2 vise principalement à recalculer une nouvelle dose journalière d'insuline pour atteindre à nouveau l'équilibre glycémique.

Le circuit de titration 30 comprend donc une règle de dosage qui réalise ce calcul lors d'une étape de titration S6 en fonction notamment de valeurs de glycémie et de paramètres physiologiques et/ou médicaux propres audit patient.

On comprendra ici que les mesures des glycémies pourront être réalisées par la sonde glycémique 40 et que les paramètres physiologiques et/ou médicaux propres au patient sont des informations préalablement stockées dans le module mémoire 10 suite par exemple à une première visite chez le médecin traitant.

Lors de cette étape de titration S6, la règle de dosage permet de :
- diminuer ladite dose journalière d'au moins une unité lorsque, pendant une période d'au moins un jour, la moyenne des glycémies mesurées est strictement inférieure à ladite borne inférieure de glycémie, et
- augmenter ladite dose journalière d'au moins une unité lorsque, pendant une période d'au moins un jour, la moyenne des glycémies mesurées est strictement supérieure à ladite borne supérieure de glycémie.

A chaque fois que la dose d'insuline journalière est calculée par le circuit de titration 30, cette information est envoyée lors d'une étape S7 à un injecteur 70.

Des moyens de communication sans fil pourront par exemple être utilisés.

L'injecteur 70 reçoit donc cette information, et le patient peut réaliser son injection d'insuline lors d'une étape S8.

Dans l'exemple décrit, il est prévu un circuit de contrôle 80 qui va recevoir de l'injecteur 70 l'information relative à la dose réellement injectée.

Ce circuit 80 va ainsi pouvoir comparer cette dose avec la dose théorique calculée par le circuit de titration 30.

Ce contrôle S9 permettra au circuit de titration 30 de réajuster par la suite la ou les doses d'insuline calculées dans les titrations suivantes.

Il est en effet possible que la dose réellement injectée soit très légèrement différente de la dose calculée.

Dans l'exemple décrit ici, le circuit de titration 30 réalise une analyse des glycémies mesurées au cours de cette phase de titration.

Cette analyse permet alors de détecter que l'équilibre glycémique est à nouveau atteint : par exemple, la dose a été stabilisée pendant plusieurs jours et les données de glycémie sont majoritairement dans l'intervalle attendue.

Dans ce cas, le circuit 30 est prévu pour émettre à son tour un signal d'équilibre.

Sur réception de ce signal, le processeur 80 va désactiver le circuit de titration 30 et va réactiver le circuit de traitement 20.

On a ainsi un procédé itératif qui est orchestré et géré par le processeur 80 et qui permet de basculer d'une phase d'équilibre vers une phase de titration en fonction de l'état glycémique du patient (équilibre ou déséquilibre).

On notera que les règles de dosage et de gestion diffèrent notamment en ce que le circuit de titration réalise son observation et son analyse des valeurs glycémiques sur une période de temps relativement courte, tandis que le circuit de traitement et l'étape d'identification de l'équilibre dans le circuit de titration se basent sur des périodes plus longues.

L'homme du métier comprendra ici que l'objectif de cette approche est de diminuer la sensibilité du mécanisme lors de la phase d'équilibre ; on parle aussi d'amortissement.

La glycémie est en effet un facteur physiologiquement instable par nature. La gestion de cette phase d'équilibre proposée dans le cadre de la présente invention permet d'endiguer les risques liés aux oscillations de cette instabilité.

En détectant en amont l'apparition d'un déséquilibre glycémique chez un patient, la présente invention anticipe l'intervention du médecin en permettant de rebasculer, sans attendre, le patient dans une phase de titration pour calculer dynamiquement une nouvelle dose d'insuline.

Préférentiellement, la bascule peut s'exécuter dans l'autre sens lorsque, par exemple, l'équilibre est à nouveau atteint.

Un tel système d'ASG permet de gérer dynamiquement l'état glycémique d'un patient et évite l'apparition des accidents glycémiques chez le patient diabétique.

On pourra enfin prévoir un mode de réalisation (non représenté ici) avec un circuit de sécurité qui permet de déclencher une diminution immédiate de la dose si la glycémie est en dessous d'un certain seuil correspondant à une hypoglycémie.

Ce circuit de sécurité peut désactiver les autres circuits et envoyer une alerte au médecin. Seul le médecin sera alors à même de réactiver l'un ou l'autre des circuits.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

Il devra également être observé que les signes de références mis entre parenthèses dans les revendications qui suivent ne présentent en aucun cas un caractère limitatif ; ces signes ont pour seul but d'améliorer l'intelligibilité et la compréhension des revendications qui suivent ainsi que la portée de la protection recherchée.

## Revendications

1. Dispositif médical (100) de gestion de l'équilibre glycémique d'un patient diabétique, ledit dispositif (100) comportant :
- un module mémoire (10) configuré pour mémoriser une pluralité de glycémies mesurées pendant une phase d'équilibre glycémique (P1) sur une période de temps déterminée, lesdites glycémies mesurées étant relatives à une teneur d'un composant sanguin représentatif du taux de glycémie dudit patient, et
- un circuit de traitement (20) mettant en œuvre une règle de gestion configurée pour détecter un état de déséquilibre glycémique chez le patient en comparant lesdites glycémies mesurées avec une plage de valeurs seuil présentant une borne supérieure de glycémie correspondant à un état de glycémie élevée et une borne inférieure de glycémie correspondant à un état de glycémie basse, ledit circuit de traitement (20) étant configuré pour émettre un signal d'avertissement lorsqu'un état de déséquilibre glycémique est détecté
- un circuit de titration (30) configuré pour calculer une dose journalière d'insuline
le dispositif comprenant en outre un processeur (80) configuré pour, sur réception du signal d'avertissement :
- désactiver ledit circuit de traitement (20) de manière à mettre fin à la phase d'équilibre (P1), et
- activer le circuit de titration (30) de manière à initier une phase de titration (P2) pour déterminer une nouvelle dose journalière d'insuline ;
ledit circuit de titration (30) étant configuré pour émettre un signal d'équilibre lorsqu'un état d'équilibre glycémique est détecté au cours de la phase de titration ;
le processeur (80) étant en outre configuré pour, sur réception du signal d'équilibre :
- activer le circuit de traitement (20), et
- désactiver le circuit de titration (30).

2. Dispositif (100) selon la revendication 1, dans lequel ladite règle de gestion comprend un algorithme configuré pour détecter un état de déséquilibre glycémique chez le patient lorsqu'un pourcentage déterminé desdites glycémies mesurées n'est pas dans ladite plage de valeurs seuil.

3. Dispositif (100) selon la revendication 1, dans lequel la règle de gestion comprend un algorithme configuré pour détecter un état de déséquilibre glycémique chez le patient lorsque la distribution desdites glycémies mesurées sur ladite période de temps présente un écart-type supérieur à une déviation seuil déterminée.

4. Dispositif selon l'une quelconque des revendications précédentes, dans lequel le circuit de titration (30) comprend une règle de dosage configurée pour calculer ladite nouvelle dose journalière d'insuline en fonction notamment de valeurs de glycémie et de paramètres physiologiques et/ou médicaux propres audit patient.

5. Dispositif (100) selon la revendication 4, comprenant un circuit de contrôle (90) configuré pour comparer ladite dose calculée avec la dose injectée dans le corps dudit patient.

6. Dispositif (100) selon la revendication 5, dans lequel ledit circuit de titration (30) comprend des moyens aptes à communiquer avec un injecteur d'insuline (40) pour récupérer les informations relatives à la dose réellement injectée dans le corps dudit patient par ledit injecteur d'insuline (40).

7. Dispositif (100) selon l'une quelconque des revendications précédentes, comprenant au moins un capteur d'activité (51, 52) configuré pour mesurer et fournir des informations relatives à l'activité dudit patient, dans lequel ladite règle de gestion est configurée pour :
- traiter lesdites informations d'activité de manière à détecter au moins un événement perturbateur relatif à un changement d'activité dudit patient, et
- détecter un état de déséquilibre glycémique chez le patient en fonction dudit au moins un événement perturbateur.

8. Dispositif (100) selon l'une quelconque des revendications précédentes, comprenant une sonde glycémique (53) configurée pour mesurer une teneur d'un composant sanguin représentatif du taux de glycémie dudit patient.

9. Dispositif (100) selon la revendication 8, dans lequel ladite sonde glycémique (53) est configurée pour réaliser ladite mesure à intervalle de temps régulier.

10. Procédé de gestion de l'équilibre glycémique d'un patient diabétique, ledit procédé mis en œuvre par un dispositif selon l'une quelconque des revendications 1 à 9 comportant les étapes suivantes :
- une mémorisation (S1) d'une pluralité de glycémies mesurées pendant une phase d'équilibre glycémique (P1) sur une période de temps déterminée, lesdites glycémies mesurées étant relatives à une teneur d'un composant sanguin représentatif du taux de glycémie dudit patient ;
- une comparaison (S2) desdites glycémies mesurées avec une plage de valeurs seuil présentant une borne supérieure de glycémie correspondant à un état de glycémie élevée et une borne inférieure de glycémie correspondant à un état de glycémie basse ;
- une détection (S3₁, S3₂, S3₃) d'un état de déséquilibre glycémique chez le patient en fonction de la mise en œuvre d'une règle de gestion analysant (S3₃) les résultats de ladite comparaison (S2) ;
- en cas de détection d'un état de déséquilibre chez le patient, une émission (S4) d'un signal d'avertissement ;
- suite à la réception dudit signal d'avertissement, une étape d'arrêt (S5₁) de la phase d'équilibre glycémique (P1) suivie d'une étape d'initialisation (S5₂) de la phase de titration (P2), ladite phase de titration (P2) comprenant une étape de titration (S6) au cours de laquelle est mise en œuvre une règle de dosage pour déterminer une nouvelle dose journalière d'insuline ;
- en cas de détection d'un état d'équilibre glycémique chez le patient lors de la phase de titration, une émission d'un signal d'équilibre ; et
- suite à la réception dudit signal d'équilibre, la désactivation du circuit de titration et l'activation du circuit de traitement.

11. Procédé selon la revendication 10, dans lequel, lors de l'étape d'analyse (S3₃), un état de déséquilibre glycémique est détecté chez le patient lorsqu'un pourcentage déterminé desdites valeurs de glycémie n'est pas dans ladite plage de valeurs seuil.

12. Procédé selon la revendication 10, dans lequel, lors de l'étape d'analyse (S3₃), un état de déséquilibre glycémique est détecté chez le patient lorsque la distribution desdites valeurs mesurées sur ladite période de temps présente un écart-type supérieur à une déviation seuil déterminée.

13. Procédé selon l'une quelconque des revendications 10 à 12, dans lequel, lors de l'étape de titration (S6), la règle de dosage calcule ladite nouvelle dose journalière d'insuline en fonction de valeurs de glycémie et de paramètres physiologiques et/ou médicaux propres audit patient.

14. Procédé selon la revendication 13, comprenant une communication des informations relatives à ladite dose réellement injectée dans le corps dudit patient par ledit injecteur d'insuline (40).

15. Procédé selon l'une quelconque des revendications 13 à 14, comprenant une étape de contrôle (S9) au cours de laquelle ladite dose calculée lors de l'étape de titration (S6) est comparée avec la dose injectée dans le corps du patient.

16. Procédé selon l'une quelconque des revendications 10 à 15, dans lequel l'étape de détection (S3₁, S3₂, S3₃) comprend :
- une mesure (S3₁) de l'activité physique dudit patient,
- une détection (S3₂) d'au moins un événement perturbateur relatif à un changement d'activité dudit patient,
dans lequel, lors de l'étape d'analyse (S3₃), ladite règle de gestion prend en considération ledit au moins un événement perturbateur pour détecter un état de déséquilibre glycémique chez le patient.

17. Procédé selon l'une quelconque des revendications 10 à 16, comprenant une mesure (S0) d'une teneur d'un composant sanguin représentatif du taux de glycémie dudit patient.

18. Procédé selon la revendication 17, dans lequel ladite mesure (S0) est réalisée à intervalle de temps régulier.

19. Programme d'ordinateur comportant des instructions adaptées pour l'exécution des étapes du procédé selon l'une quelconque des revendications 10 à 18 lorsque ledit programme d'ordinateur est exécuté par au moins un processeur.

## Patentansprüche

1. Medizinische Vorrichtung (100) für das Management des Blutzuckergleichgewichts eines Patienten mit Diabetes, wobei die Vorrichtung (100) umfasst:
- ein Speichermodul (10), welches dazu konfiguriert ist, eine Vielzahl von Blutzuckerwerten zu speichern, die während einer Phase des Blutzuckergleichgewichts (P1) über einen bestimmten Zeitraum gemessen wurden, wobei sich die gemessenen Blutzuckerwerte auf einen Gehalt eines Blutbestandteils beziehen, der für den Blutzuckerspiegel des Patienten repräsentativ ist, und
- eine Verarbeitungsschaltung (20), die eine Managementregel implementiert, welche dazu konfiguriert ist, einen Zustand eines Blutzuckerungleichgewichts bei dem Patienten zu erkennen, indem die gemessenen Blutzuckerwerte mit einem Schwellenwertbereich verglichen werden, der einen oberen Blutzuckerschwellenwert aufweist, welcher einem hohen Blutzuckerwert entspricht, und einen unteren Blutzuckerschwellenwert aufweist, welcher einem niedrigen Blutzuckerspiegel entspricht,
wobei die Verarbeitungsschaltung (20) dazu konfiguriert ist, ein Warnsignal auszugeben, wenn ein Zustand des Blutzuckerungleichgewichts erkannt wird,
- eine Titrationsschaltung (30), die dazu konfiguriert ist, eine Tagesdosis Insulin zu berechnen,
wobei das Gerät ferner einen Prozessor (80) umfasst, der dazu konfiguriert ist, bei Empfang des Warnsignals:
- die Verarbeitungsschaltung (20) zu deaktivieren, um die Gleichgewichtsphase (P1) zu beenden, und
- die Titrationsschaltung (30) zu aktivieren, um eine Titrationsphase (P2) einzuleiten, um eine neue Tagesdosis Insulin zu bestimmen,
wobei die Titrationsschaltung (30) dazu konfiguriert ist, ein Gleichgewichtssignal auszugeben, wenn während der Titrationsphase ein Zustand des Blutzuckergleichgewichts erfasst wird,
wobei der Prozessor (80) ferner dazu konfiguriert ist, bei Empfang des Gleichgewichtssignals:
- die Verarbeitungsschaltung (20) zu aktivieren, und
- die Titrationsschaltung (30) zu deaktivieren.

2. Vorrichtung (100) nach Anspruch 1, wobei die Managementregel einen Algorithmus umfasst, der dazu konfiguriert ist, einen Zustand des Blutzuckerungleichgewichts bei dem Patienten zu erkennen, wenn ein festgelegter Prozentsatz der gemessenen Blutzuckerwerte nicht innerhalb des Schwellenwertbereichs liegt.

3. Vorrichtung (100) nach Anspruch 1, wobei die Managementregel einen Algorithmus umfasst, der dazu konfiguriert ist, einen Zustand des Blutzuckerungleichgewichts bei dem Patienten zu erkennen, wenn die Verteilung der gemessenen Blutzuckerwerte über den genannten Zeitraum eine Standardabweichung aufweist, die größer ist als eine festgelegte Schwellenabweichung.

4. Vorrichtung nach einem der vorhergehenden Ansprüche, wobei die Titrationsschaltung (30) eine Dosierungsregel umfasst, die dazu konfiguriert ist, die neue Tagesdosis Insulin insbesondere abhängig von den Blutzuckerwerten und den für den Patienten spezifischen physiologischen und/oder medizinischen Parametern, zu berechnen.

5. Vorrichtung (100) nach Anspruch 4, welche eine Steuerschaltung (90) umfasst, die dazu konfiguriert ist, die berechnete Dosis mit der in den Körper des Patienten injizierten Dosis zu vergleichen.

6. Vorrichtung (100) nach Anspruch 5, wobei die Titrationsschaltung (30) Mittel umfasst, die dazu geeignet sind, mit einer Insulininjektionsvorrichtung (40) zu kommunizieren, um Informationen abzurufen, die sich auf die von der Insulininjektionsvorrichtung (40) tatsächlich in den Körper des Patienten injizierte Dosis beziehen.

7. Vorrichtung (100) nach einem der vorhergehenden Ansprüche, welche wenigstens einen Aktivitätssensor (51, 52) umfasst, der dazu konfiguriert ist, Informationen zur Aktivität des Patienten zu messen und bereitzustellen, wobei die Managementregel dazu konfiguriert ist:
- diese Aktivitätsinformationen zu verarbeiten, um wenigstens ein störendes Ereignis in Bezug auf eine Änderung der Aktivität des Patienten zu erfassen, und
- einen Zustand eines Blutzuckerungleichgewichts in dem Patienten abhängig von dem wenigstens einen, störenden Ereignis zu erfassen.

8. Vorrichtung (100) nach einem der vorhergehenden Ansprüche, umfassend einen Blutzuckersensor (53), der dazu konfiguriert ist, einen Gehalt eines Blutbestandteils (30) zu messen, der für den Blutzuckerspiegel des Patienten repräsentativ ist.

9. Vorrichtung (100) nach Anspruch 8, wobei der Blutzuckersensor (53) dazu konfiguriert ist, die Messung in regelmäßigen zeitlichen Abständen durchzuführen.

10. Verfahren für das Management des Blutzuckergleichgewichts eines Patienten mit Diabetes, wobei das durch eine Vorrichtung nach einem der Ansprüche 1 bis 9 implementierte Verfahren die folgenden Schritte umfasst:
- Speichern (S1) einer Vielzahl von Blutzuckerwerten, die während einer Blutzuckergleichgewichtsphase (P1) über einen bestimmten Zeitraum gemessen wurden, wobei sich die gemessenen Blutzuckerwerte auf einen Gehalt eines Blutbestandteils beziehen, der für den Blutzuckerspiegel des Patienten repräsentativ ist,
- Vergleichen (S2) der gemessenen Blutzuckerwerte mit einem Schwellenwertbereich, der einen oberen Blutzuckerschwellenwert aufweist, welcher einem hohen Blutzuckerspiegel entspricht, und einen unteren Blutzuckerschwellenwert aufweist, welcher einem niedrigen Blutzuckerspiegel entspricht,
- Erkennen (S3₁, S3₂, S3₃) eines Zustands eines Blutzuckerungleichgewichts bei dem Patienten abhängig von der Implementierung einer Managementregel, die die Ergebnisse des Vergleichs (S2) analysiert (S3₃),
- wenn bei dem Patienten ein Ungleichgewichtszustand erkannt wird, Ausgeben (S4) eines Warnsignals,
- nach dem Empfang des Warnsignals einen Schritt des Anhaltens (S5₁) der Blutzuckergleichgewichtsphase (P1), gefolgt von einem Schritt (S5₂) des Initialisierens der Titrationsphase (P2), wobei die Titrationsphase (P2) einen Titrationsschritt (S6) umfasst, in dessen Verlauf eine Dosierungsregel implementiert wird, um eine neue Tagesdosis Insulin zu bestimmen,
- wenn bei dem Patienten während der Titrationsphase ein Zustand des Blutzuckergleichgewichts erkannt wird, Ausgeben eines Gleichgewichtssignals, und
- nach dem Empfang des Gleichgewichtssignals, Inaktivieren der Titrationsschaltung und Aktivieren der Verarbeitungsschaltung.

11. Verfahren nach Anspruch 10, wobei im Analyseschritt (S3₃) ein Zustand des Blutzuckerungleichgewichts bei dem Patienten erkannt wird, wenn ein festgelegter Prozentsatz der Blutzuckerwerte nicht in dem Schwellenwertbereich liegt.

12. Verfahren nach Anspruch 10, wobei im Analyseschritt (S3₃) ein Zustand des Blutzuckerungleichgewichts bei dem Patienten erkannt wird, wenn die Verteilung der Messwerte über den genannten Zeitraum eine Standardabweichung aufweist, die größer ist als eine festgelegte Schwellenwertabweichung.

13. Verfahren nach einem der Ansprüche 10 bis 12, wobei im Titrationsschritt (S6) die Dosierungsregel die neue Tagesdosis Insulin abhängig von den Blutzuckerwerten und den für den Patienten spezifischen physiologischen und/oder medizinischen Parametern berechnet.

14. Verfahren nach Anspruch 13, welches eine Übermittlung von Informationen bezüglich der durch die Insulininjektionsvorrichtung (40) tatsächlich in den Körper des Patienten injizierten Dosis umfasst.

15. Verfahren nach einem der Ansprüche 13 bis 14, welches einen Kontrollschritt (S9) umfasst, in dem die im Titrationsschritt (S6) berechnete Dosis mit der in den Körper des Patienten injizierten Dosis verglichen wird.

16. Verfahren nach einem der Ansprüche 10 bis 15, wobei der Erkennungsschritt (S3₁, S3₂, S3₃) umfasst:
- eine Messung (S3₁) der körperlichen Aktivität des Patienten,
- eine Erfassung (S3₂) wenigstens eines störenden Ereignisses, bezogen auf eine Änderung der Aktivität des Patienten,
wobei in dem Analyseschritt (S3₃) die Managementregel das wenigstens eine störende Ereignis berücksichtigt, um einen Zustand des Blutzuckerungleichgewichts bei dem Patienten zu erkennen.

17. Verfahren nach einem der Ansprüche 10 bis 16, welches eine Messung (S0) eines Gehalts eines Blutbestandteils umfasst, der für den Blutzuckerspiegel des Patienten repräsentativ ist.

18. Verfahren nach Anspruch 17, wobei die Messung (S0) in regelmäßigen zeitlichen Abständen durchgeführt wird.

19. Computerprogramm mit Anweisungen, die dazu ausgebildet sind, die Schritte des Verfahrens nach einem der Ansprüche 10 bis 18 auszuführen, wenn das Computerprogramm von wenigstens einem Prozessor ausgeführt wird.

## Claims

1. Medical device (100) for managing the glycaemic balance of a diabetic patient, said device (100) comprising:
- a memory module (10) configured for storing a plurality of blood glucose levels measured during a glycaemic balance phase (P1) over a given period of time, said blood glucose levels measured being relative to a content of a blood component representative of the blood glucose level of said patient, and
- a processing circuit (20) implementing a management rule configured for detecting a glycaemic imbalance state in the patient by comparing said blood glucose levels measured with a threshold value range having an upper blood glucose limit corresponding to a high blood glucose state and a lower blood glucose limit corresponding to a low blood glucose state,
said processing circuit (20) being configured for emitting a warning signal when a glycaemic imbalance state is detected,
- a titration circuit (30) configured for calculating a daily insulin dose;
the device further comprising a processor (80) configured for, upon receipt of the warning signal:
- deactivating said processing circuit (20) so as to bring an end to the balance phase (P1), and
- activate the titration circuit (30) so as to initiate a titration phase (P2) for determining a new daily insulin dose;
said titration circuit (30) being configured for emitting a balance signal when a glycaemic balance state is detected during the titration phase;
the processor (80) being further configured for, upon receipt of the balance signal:
- activate the processing circuit (20), and
- deactivate the titration circuit (30).

2. Device (100) according to Claim 1, in which said management rule comprises an algorithm configured for detecting a glycaemic imbalance state in the patient when a given percentage of said blood glucose levels measuresd is not in said threshold value range.

3. Device (100) according to Claim 1, in which the management rule comprises an algorithm configured for detecting a glycaemic imbalance state in the patient when the distribution of said blood glucose levels measured over said period of time has a standard deviation above a given threshold deviation.

4. Device according to any one of the preceding claims, in which the titration circuit (30) is capable of implementing a dosage rule configured for calculating said new daily insulin dose as a function in particular of blood glucose values and of physiological and/or medical parameters specific to said patient.

5. Device (100) according to Claim 4, comprising a control circuit (90) configured for comparing said calculated dose with the dose injected into the body of said patient.

6. Device (100) according to Claim 5, in which said titration circuit (30) is capable of communicating with an insulin injector (40) in order to recover the information relating to the dose actually injected into the body of said patient by said insulin injector (40).

7. Device (100) according to any one of the preceding claims, comprising at least one activity sensor (51, 52) configured for measuring and supplying information relating to the activity of said patient, in which said management rule is configured for:
- processing said activity information so as to detect at least one disrupting event relating to a change in activity of said patient, and
- detecting a glycaemic imbalance state in the patient as a function of said at least one disrupting event.

8. Device (100) according to any one of the preceding claims, comprising a glycaemic probe (53) configured for measuring a content of a blood component representative of the blood glucose level of said patient.

9. Device (100) according to Claim 8, in which said glycaemic probe (53) is configured for carrying out said measurement at regular time intervals.

10. Method for managing the glycaemic balance of a diabetic patient, said method carried out by a device according to any one of the claims 1 to 9 comprising the following steps:
- storage (S1) of a plurality of blood glucose levels measured during a glycaemic balance phase (P1) over a given period of time, said blood glucose levels measured being relative to a content of a blood component representative of the blood glucose level of said patient;
- comparison (S2) of said blood glucose levels measured with a threshold value range having an upper blood glucose limit corresponding to a high blood glucose state and a lower blood glucose level corresponding to a low blood glucose state;
- detection (S3₁, S3₂, S3₃) of a glycaemic imbalance state in the patient as a function of the implementation of a management rule analysing (S3₃) the results of said comparison (S2);
- in the event of detection of an imbalance state in the patient, emission (S4) of a warning signal;
- following the receipt of said warning signal, a step of stopping (S5₁) the glycaemic balance phase (P1), followed by a step of initializing (S5₂) the titration phase (P2), said titration phase (P2) comprising a titration step (S6) during which a dosage rule is implemented for determining a new daily insulin dose;
- when a glycaemic balance state is detected in the patient during the titration phase, emission of a balance signal; and
- upon receipt of the balance signal, the deactivation of the titration circuit and the activation of the processing circuit.

11. Method according to Claim 10, in which, during the analysis step (S3₃), a glycaemic imbalance state is detected in the patient when a given percentage of said blood glucose values is not in said threshold value range.

12. Method according to Claim 10, in which, during the analysis step (S3₃), a glycaemic imbalance state is detected in the patient when the distribution of said values measured over said period of time has a standard deviation above a given threshold deviation.

13. Method according to any one of the claims 10 to 12, in which, during the titration step (S6), the dosage rule calculates said new daily insulin dose as a function of blood glucose values and of physiological and/or medical parameters specific to said patient.

14. Method according to Claims 13, comprising a communication of the information relating to said dose actually injected into the body of said patient by said insulin injector (40).

15. Method according to any one of Claims 13 to 14, comprising a control step (S9) during which said dose calculated during the titration step (S6) is compared with the dose injected into the body of the patient.

16. Method according to any one of Claims 10 to 15, in which the detection step (S3₁, S3₂, S3₃) comprises:
- measurement (S3₁) of the physical activity of said patient,
- detection (S3₂) of at least one disrupting event relating to a change in activity of said patient,
in which, during the analysis step (S3₃), said management rule takes into consideration said at least one disrupting event for detecting a glycaemic imbalance state in the patient.

17. Method according to any one of Claims 10 to 16, comprising a measurement (S0) of a content of a blood component representative of the blood glucose level of said patient.

18. Method according to Claim 17, in which said measurement (S0) is carried out at regular time intervals.

19. Computer program comprising instructions suitable for executing the steps of the method according to any one of Claims 10 to 18 when said computer program is executed by at least one processor.
